# EUROPÄISCHE PATENTANMELDUNG

(11) **EP 2 143 744 A1**
(43) Veröffentlichungstag der Anmeldung: **13.01.2010**
(21) Anmeldenummer: 08012372.2
(22) Anmeldetag: 09.07.2008
(51) Int. Cl.: C08G 18/10, C08G 18/48, C08G 18/75

(54) **Hydrophile, aliphatische Polyurethan-Schäume**

(71) Anmelder: Bayer MaterialScience AG, 51368 Leverkusen (DE)
(72) Erfinder: Schönberger, Jan, Dr., 42655 Solingen (DE); Köhler, Burkhardt, Dr., 34289 Zierenberg (DE); Haas, Peter. Dr., 42781 Haan (DE)

(57) **Zusammenfassung**

Die Erfindung betrifft hydrophile, aliphatische Polyurethan-Schäume, die durch Reaktion von speziellen monomerenarmen Präpolymeren und gegebenenfalls oligomeren Isocyanaten in Gegenwart von Wasser und Katalysatoren zugänglich sind. Aufgrund ihrer Absorptionseigenschaften sind die hydrophilen Polyurethan-Schäume insbesondere geeignet zur Herstellung von Wundauflagen, kosmetischen Artikeln oder Inkontinenzprodukten.

## Beschreibung

Die Erfindung betrifft hydrophile, aliphatische Polyurethan-Schäume, die durch Reaktion von speziellen monomerenarmen Präpolymeren und gegebenenfalls oligomeren Isocyanaten in Gegenwart von Wasser und Katalysatoren zugänglich sind. Aufgrund ihrer Absorptionseigenschaften sind die hydrophilen Polyurethan-Schäume insbesondere geeignet zur Herstellung von Wundauflagen, kosmetischen Artikeln oder Inkontinenzprodukten.

Die EP-A 949285 beschreibt die Reaktion von Polyisocyanaten mit primären Diaminen, niedermolekularen Polyolen und hochmolekularen Polyolen. Bei dieser Reaktion ist nicht auszuschließen, dass erhebliche Teile der isocyanatreaktiven Substanzen nicht umgesetzt werden und anschließend aus dem hydrophilen Schaum extrahierbar sind.

Die GB 1571730 beschreibt die Reaktion von Diisocyanaten mit hohem Dampfdruck wie Isophorondiisocyanat (IPDI) und Bis(isocyanatocyclohexyl)methan (HMDI) mit Polyolen. Auch hier bleiben nicht umgesetzte Komponenten zurück. Ferner ist das Arbeiten mit freien, nicht derivatisierten Diisocyanaten aus Sicht der Arbeitshygiene problematisch. In der WO 2004013215 werden ebenfalls leicht flüchtige Diisocyanate umgesetzt.

In der GB 1571730 wie auch in der US 3778390, US 3799898 und FR 2077388 werden als Schaumstabilisatoren siliziumhaltige und siliziumfreie nichtionische, Sulfat-, Phosphat- und Sulfonat-Emulgatoren genannt. Diese weisen jedoch eine geringe Zellverträglichkeit auf. Die Verwendung von Carboxylaten wird nicht erwähnt.

Die WO2003/097727, US 5065752 und US 5064653 beschreiben die Schaumbildungsreaktion von Präpolymeren in Gegenwart von Acrylamid-Acrylsäure-Copolymeren. Diese Produkte sind chemisch nicht angekoppelt und können komplett extrahiert werden, was ebenfalls nicht wünschenswert ist.

In der US 3903232 und US 388941 werden Präpolymere mit Polyethern umgesetzt. Auch hier besteht die Gefahr des Auftretens nicht angekoppelter Polyole. Die US 5296518 beschreibt ebenso die Umsetzung von Präpolymeren mit Polyethern, wobei drei unterschiedliche Polyole eingesetzt werden, was die Wirtschaftlichkeit dieses Verfahrens in Frage stellt. Zudem lässt sich mit dem dort beschriebenen Verfahren nicht sicherstellen, dass keine niedermolekularen Isocyanate im Gemisch verbleiben, was nicht wünschenswert ist. Die Verwendung von Carboxylaten wird nicht erwähnt. Die Herstellung der Präpolymere erfordert zumeist unwirtschaftlich lange Reaktionszeiten.

Aufgabe der vorliegenden Erfindung war daher die Bereitstellung eines Verfahrens zur Herstellung hydrophiler, aliphatischer Polyurethan-Schäume, welche insbesondere als Bestandteil einer Wundauflage, eines kosmetischen Artikels oder eines Inkontinenzprodukts eingesetzt werden können, und daher nur wenig extrahierbare Bestandteile aufweisen sollen. Zudem ist es von großer prozesstechnischer Wichtigkeit, dass die Polyurethan-Schäume nach dem Aufschäumen keinen Volumenschrumpf erleiden. Überdies sollen bei deren Herstellung nur Polyisocyanate mit einem niedrigen Dampfdruck, also keine unmodifizierten Diisocyanate, eingesetzt werden. Die hydrophilen, aliphatischen Polyurethan-Schäume sollen überdies eine rasche und hohe Absorption von physiologischer Salzlösung bzw. von Wundflüssigkeit aufweisen, ohne dass hierzu zwingend superabsorbierende Polymere eingesetzt werden müssen. Wundauflagen aus diesen Polyurethan-Schäumen sollen zellverträglich sein (nicht cytotoxisch) und sich in der Anwendung der Wundform optimal anpassen.

Es wurde nun gefunden, dass sich Präpolymere aus aliphatischen Diisocyanaten, vorzugsweise HDI, und Polyethern mit einem Ethylenoxid-Gehalt von mindestens 50 mol% bezogen auf den Gesamtgehalt an Oxyalkyleneinheiten in Mischungen mit Uretdiongruppen- und Isocyanuratgruppen-haltigen Oligomeren auf Basis von Hexamethylendiisocyanat (HDI) mit Wasser in Gegenwart ausgesuchter Aktivatoren und gegebenenfalls Schaumstabilisatoren verschäumen lassen.

Gegenstand der vorliegenden Erfindung ist daher ein Verfahren zur Herstellung hydrophiler, aliphatischer Polyurethan-Schäume, bei dem Zusammensetzungen umfassend
A) isocyanatfunktionelle Präpolymere mit einem Gewichtsanteil an niedermolekularen, aliphatischen Diisocyanaten mit einer Molmasse von 140 bis 278 g/mol von unter 1,0 Gew.-% bezogen auf das Präpolymer, erhältlich durch Umsetzung von
   A1) niedermolekularen, aliphatischen Diisocyanaten einer Molmasse von 140 bis 287 g/mol mit
   A2) di- bis hexafunktionellen, bevorzugt tri- bis hexafunktionellen Polyalkylenoxiden einer OH-Zahl von 22,5 bis 112, bevorzugt von 31,5 bis 56, und einem Ethylenoxidanteil von 50 bis 100 mol%, bevorzugt 60 bis 85 mol%, bezogen auf die Gesamtmenge der enthaltenen Oxyalkylengruppen,
B) gegebenenfalls heterocyclische, 4-Ring- oder 6-Ring-Oligomere von niedermolekularen, aliphatischen Diisocyanaten mit einer Molmasse von 140 bis 278 g/mol,
C) Wasser,
D) gegebenenfalls Katalysatoren,
E) C₈- bis C₂₂-Monocarbonsäuren oder deren Ammonium- oder Alkalisalze oder C₁₂- bis C₄₄-Dicarbonsäuren oder deren Ammonium- oder Alkalisalze,
F) gegebenenfalls Tenside und
G) gegebenenfalls ein- oder mehrwertige Alkohole
   bereitgestellt, aufgeschäumt und ausgehärtet werden.

Bevorzugt weisen die in A) eingesetzten Präpolymere einen Restmonomergehalt von unter 0,5 Gew.-% bezogen auf das Präpolymer auf. Dieser Gehalt kann durch entsprechend gewählte Einsatzmengen von A1) und A2) erreicht werden. Bevorzugt ist jedoch der Einsatz des Isocyanats A1) im Überschuss und anschließender, bevorzugt destillativer, Abtrennung nicht umgesetzter Monomere.

Die Herstellung der isocyanatfunktionellen Präpolymere der Komponente A) erfolgt typischerweise durch Umsetzung von einem Val der Polyolkomponente A2) mit einem bis 20 Mol, bevorzugt einem bis 10 Mol, besonders bevorzugt 5 bis 10 Mol, des niedermolekularen, aliphatischen Diisocyanats A1).

Die Umsetzung kann in Gegenwart von Urethanisierungskatalysatoren wie Zinnverbindungen, Zinkverbindungen, Aminen, Guanidinen oder Amidinen, oder in Gegenwart von Allophanatisierungskatalysatoren wie Zinkverbindungen erfolgen.

Die Umsetzung erfolgt typischerweise bei 25 bis 140°C, bevorzugt 60 bis 100°C.

Wurde mit überschüssigem Isocyanat gearbeitet so erfolgt anschließend die Entfernung des Überschusses an niedermolekularem, aliphatischen Diisocyanat bevorzugt durch Dünnschichtdestillation.

Vor, während und nach der Reaktion oder destillativen Abtrennung des Diisocyanatüberschusses können saure oder alkylierende Stabilisatoren, wie Benzoylchlorid, Isophthaloylchlorid, Methyltosylat, Chlorpropionsäure, HCI oder Antioxidantien, wie Di-*tert*-butylkresol oder Tocopherol zugesetzt werden.

Der NCO-Gehalt der isocyanatfunktionellen Präpolymere A) beträgt bevorzugt 1,5 bis 4,5 Gew.-%, besonders bevorzugt 1,5 bis 3,5 Gew.-% und ganz besonders bevorzugt 1,5 bis 3,0 Gew.-%.

Beispiele für niedermolekulare, aliphatischen Diisocyanate der Komponente A1) sind Hexamethylendiisocyanat (HDI), Isophorondiisocyanat (IPDI), Butylendiisocyanat (BDI), Bisisocyanatocyclohexylmethan (HMDI), 2,2,4-Trimethylhexamethylendiisocyanat, Bisisocyanatomethylcyclohexan, Bisisocyanatomethyltricyclodecan, Xylendiisocyanat, Tetramethylxylylendiisocyanat, Norbornandiisocyanat, Cyclohexandiisocyanat oder Diisocyanatododecan, wobei Hexamethylendiisocyanat (HDI), Isophorondiisocyanat (IPDI), Butylendiisocyanat (BDI) und Bis(isocyanatocyclohexyl)methan (HMDI) bevorzugt sind. Besonders bevorzugt sind BDI, HDI, IPDI, ganz besonders bevorzugt Hexamethylendiisocyanat und Isophorondiisocyanat.

Polyalkylenoxide der Komponente A2) sind bevorzugt Copolymere aus Ethylenoxid und Propylenoxid mit einem Ethylenoxidgehalt, bezogen auf die Gesamtmenge der enthaltenen Oxyalkylengruppen, von 50 bis 100 mol%, bevorzugt 60 bis 85 mol%, gestartet auf Polyolen oder Aminen. Geeignete Starter dieser Art sind Glycerin, Trimethylolpropan (TMP), Sorbit, Pentaerythrit, Triethanolamin, Ammoniak oder Ethylendiamin

Die Polyalkylenoxide der Komponente A2) besitzen typischerweise zahlenmittlere Molekulargewichte von 1000 bis 15000 g/mol, bevorzugt von 3000 bis 8500 g/mol.

Ferner besitzen die Polyalkylenoxide der Komponente A2) OH-Funktionalitäten von 2 bis 6, bevorzugt von 3 bis 6, besonders bevorzugt von 3 bis 4.

Gegebenenfalls einzusetzende Verbindungen der Komponente B) sind heterocyclische, 4-Ring-oder 6-Ring-Oligomere von niedermolekularen, aliphatischen Diisocyanaten mit einer Molmasse von 140 bis 278 g/mol wie Isocyanurate, Iminooxadiazindione oder Uretdione der vorgenannten niedermolekularen, aliphatischen Diisocyanate. Bevorzugt sind heterocyclische 4-Ring-Oligomere wie Uretdione.

Der durch Einsatz der Komponente B) erhöhte Gehalt an Isocyanatgruppen sorgt für ein besseres Aufschäumen durch mehr gebildetes CO₂ aus der Isocyanat-Wasser-Reaktion.

Das als Komponente C) einzusetzende Wasser kann als solches, als Kristallwasser eines Salzes, als Lösung in einem dipolar-aprotischen Lösungsmittel oder auch als Emulsion eingesetzt werden. Bevorzugt wird das Wasser als solches oder in einem dipolar-aprotischen Lösungsmittel eingesetzt. Ganz besonders bevorzugt wird das Wasser als solches eingesetzt.

Zur Beschleunigung der Urethanbildung können in Komponente D) Katalysatoren eingesetzt werden. Dabei handelt es sich typischerweise um die dem Fachmann aus der Polyurethantechnologie bekannten Verbindungen. Bevorzugt sind hier Verbindungen der Gruppe bestehend aus katalytisch aktiven Metallsalzen, Aminen, Amidinen und Guanidinen. Beispielhaft zu nennen sind Zinndibutyldilaurat (DBTL), Zinnoctoat (SO), Zinnacetat, Zinkoctoat (ZO), 1,8-Diazabicyclo[5.4.0]undecen-7 (DBU), 1,5-Diazabicyclo[4.3.0]nonen-5 (DBN), 1,4-Diazabicyclo[3.3.0]octen-4 (DBO), N-Ethylmorpholin (NEM), Triethylendiamin (DABCO), Pentamethylguanidin (PMG), Tetrametylguanidin (TMG), Cyclotetramethylguanidin (TMGC), n-Decyltetramethylguanidin (TMGD), n-Dodecyltetramethylguanidin (TMGDO), Dimethylaminoethyltetramethylguanidin (TMGN), 1,1,4,4,5,5-Hexamethylisobiguanidin (HMIB), Phenyltetramethylguanidin (TMGP) und Hexamethylenoctamethylbiguanidin (HOBG).

Besonders bevorzugt ist der Einsatz von Aminen, Amidinen, Guanidinen oder deren Mischungen als Katalysatoren der Komponente D). Ganz besonders bevorzugt wird 1,8-Diazabicyclo[5.4.0]undecen-7 (DBU) eingesetzt.

In einer bevorzugten Ausführungsform der Erfindung werden in Komponente D) Verbindungen der vorgenannten Art als Katalysatoren eingesetzt.

Als Komponente E) werden Ammonium- und Alkalisalze von C₈- bis C₂₂-Monocarboxylaten oder deren freien Carbonsäuren oder C₁₂- bis C₄₄-Dicarboxylaten oder deren freien Dicarbonsäuren, bevorzugt Kalium- oder Natriumsalze von C₈- bis C₂₂-Monocarboxylaten oder C₁₂- bis C₄₄-Dicarboxylaten und besonders bevorzugt Natriumsalze von C₈- bis C₂₂-Monocarboxylaten eingesetzt.

Beispielsweise geeignete Verbindungen der Komponente E) sind die Ammonium-, Na-, Li- oder K-Salze von Ethylhexansäure, Octansäure, Decansäure, Dodecansäure, Palmitinsäure, Stearinsäure, den Octadecensäuren, den Octadecadiensäuren, den Octadecatriensäuren, Isostearinsäure, Erucasäure, Abietinsäure und ihren Hydrierungsprodukten. Beispiele für C₁₂- bis C₄₄-Dicarbonsäuren bzw. die daraus abgeleiteten Ammonium- und Alkalisalze sind Dodecandisäure, Dodecenyl-, Tetradecenyl-, Hexadecenyl- und Octadecenyl-Bernsteinsäure, C₃₆- und C₄₄-Dimerfettsäuren und ihre Hydrierungsprodukte sowie die entsprechenden Ammonium-, Na-, Li- oder K-Salze dieser Dicarbonsäuren.

Zur Verbesserung der Schaumbildung, Schaumstabilität oder der Eigenschaften des resultierenden Polyurethan-Schaums können Verbindungen der Komponente F) eingesetzt werden, wobei solche Additive grundsätzlich alle an sich bekannten anionischen, kationischen, amphoteren und nichtionischen Tenside sowie Mischungen hieraus sein können. Bevorzugt werden Alkylpolyglycoside, EO/PO-Blockcopolymere, Alkyl- oder Arylalkoxylate, Siloxanalkoxylate, Ester der Sulfobernsteinsäure und/oder Alkali- oder Erdalkalimetallalkanoate eingesetzt. Besonders bevorzugt werden EO/PO-Blockcopolymere eingesetzt. Bevorzugt werden allein die EO/PO-Blockcopolymere als Komponente F) eingesetzt.

Zudem können zur Verbesserung der Schaumeigenschaften des resultierenden Polyurethan-Schaums Verbindungen der Komponente G) eingesetzt werden. Hierbei handelt es sich um grundsätzlich alle dem Fachmann an sich bekannten ein- und mehrwertigen Alkohole sowie Mischungen hieraus.

Dies sind ein- oder mehrwertige Alkohole oder Polyole, wie Ethanol, Propanol, Butanol, Decanol, Tridecanol, Hexadecanol, Ethylenglykol, Neopentylglykol, Butandiol, Hexandiol, Decandiol, Trimethylolpropan, Glycerin, Pentaerythrit, monofunktionelle Polyetheralkohole und Polyesteralkohole, Polyetherdiole und Polyesterdiole.

Typischerweise werden die Komponenten A) bis G) in folgenden Mengen eingesetzt:
A) 100 Gewichtsteile isocyanatfunktioneller Präpolymere A)
B) 0 bis 30 Gewichtsteile heterocyclischer Oligomere B)
C) 0,1 bis 20 Gewichtsteile Wasser
D) 0 bis 1 Gewichtteile an Katalysatoren
E) 0,01 bis 5 Gewichtteile an C₈- bis C₁₂-Monocarbonsäuren oder deren Ammonium- oder Alkalisalze oder C₁₂- bis C₄₄-Dicarbonsäuren oder deren Ammonium- oder Alkalisalze
F) 0 bis 10 Gewichtsteile an Tensiden F)
G) 0 bis 20 Gewichtsteile an Alkoholen G)

Bevorzugt werden die Komponenten A) bis G) in folgenden Mengen eingesetzt:
A) 100 Gewichtsteile isocyanatfunktioneller Präpolymere A)
B) 1 bis 30 Gewichtsteile heterocyclischer Oligomere B)
C) 0,1 bis 20 Gewichtsteile Wasser
D) 0,01 bis 1 Gewichtteile an Katalysatoren
E) 0,01 bis 5 Gewichtteile an C₈- bis C₁₂-Monocarbonsäuren oder deren Ammonium- oder Alkalisalze oder C₁₂- bis C₄₄-Dicarbonsäuren oder deren Ammonium- oder Alkalisalze
F) 0 bis 5 Gewichtsteile an Tensiden
G) 0 bis 10 Gewichtsteile an Alkoholen G)

Besonders bevorzugt werden die Komponenten A) bis G) in folgenden Mengen eingesetzt:
A) 100 Gewichtsteile isocyanatfunktioneller Präpolymere A)
B) 5 bis 15 Gewichtsteile heterocyclischer Oligomere B)
C) 1 bis 10 Gewichtsteile Wasser
D) 0,1 bis 0,5 Gewichtteile an Katalysatoren
E) 0,1 bis 1 Gewichtteile an C₈- bis C₁₂-Monocarbonsäuren oder deren Ammonium- oder Alkalisalze oder C₁₂- bis C₄₄-Dicarbonsäuren oder deren Ammonium- oder Alkalisalze

Die Herstellung der erfindungsgemäßen hydrophilen, aliphatischen Polyurethan-Schäume erfolgt durch Mischen der Komponenten A), C), E) und gegebenenfalls B), D), F), G) in beliebiger Reihenfolge, Aufschäumen der Mischung und Aushärtung bevorzugt durch chemische Vernetzung. Bevorzugt werden die Komponenten A) und B) miteinander vorvermischt. Die Carboxylate E) und gegebenenfalls die Tenside F) werden in Form ihrer wässrigen Lösungen dem Reaktionsgemisch zugesetzt.

Das Aufschäumen kann dabei grundsätzlich durch das bei der Reaktion der Isocyanatgruppen mit Wasser gebildete Kohlendioxid erfolgen, die Verwendung von weiteren Treibmitteln ist jedoch ebenfalls möglich. So können prinzipiell auch Treibmittel aus der Klasse der Kohlenwasserstoffe wie C₃-C₆-Alkane, z.B. Butane, *n*-Pentan, *iso*-Pentan, *cyclo*-Pentan, Hexane o.ä. oder halogenierte Kohlenwasserstoffe wie Dichlormethan, Dichlormonofluormethan, Chlordifluorethane, 1,1-Dichlor-2,2,2-Trifluorethan, 2,2-Dichlor-2-fluorethan, insbesondere chlorfreie Fluorkohlenwasserstoffe wie Difluormethan, Trifluormethan, Difluorethan, 1,1,1,2-Tetrafluorethan, Tetrafluorethan (R 134 oder R 134a), 1,1,1,3,3-Pentafluorpropan (R 245 fa), 1,1,1,3,3,3-Hexafluorpropan (R 256), 1,1,1,3,3-Pentafluorbutan (R 365 mfc), Heptafluorpropan oder auch Schwefelhexafluorid verwendet werden. Auch Gemische dieser Treibmittel sind verwendbar.

Die anschließende Aushärtung erfolgt typischerweise bei Raumtemperatur.

Ein weiterer Gegenstand der vorliegenden Erfindung sind die erfindungsgemäßen Zusammensetzungen sowie daraus erhältliche hydrophile, aliphatische Polyurethan-Schäume.

Gegenstand der vorliegenden Erfindung sind überdies die nach dem erfindungsgemäßen Verfahren hergestellten Polyurethan-Schäume, sowie die Verwendung der hydrophilen, aliphatischen Polyurethan-Schäume als Weichschaum, als Bestandteil einer Wundauflage, eines kosmetischen Artikels oder eines Inkontinenzprodukts. Bevorzugt ist jedoch die Verwendung der Polyurethan-Schäume als Bestandteil einer Wundauflage, eines kosmetischen Artikels oder eines Inkontinenzprodukts, besonders bevorzugt ist die Verwendung als Bestandteil einer Wundauflage, ganz besonders bevorzugt als Wundauflage mit direktem Haut- und Wundkontakt der menschlichen oder tierischen Haut.

Die Polyurethan-Schäume weisen eine poröse, zumindest teilweise offenzellige Struktur mit miteinander kommunizierenden Zellen auf. Die Dichte der Polyurethan-Schäume liegt dabei typischerweise bei 0,01 bis 0,5 g/cm³, bevorzugt liegt sie bei 0,02 bis 0,4 g/cm³, besonders bevorzugt bei 0,05 bis 0,3 g/cm³ und ganz besonders bevorzugt bei 0,1 bis 0,2 g/cm³ (Bestimmung nach DIN 53420).

Das Absorptionsvermögen gegenüber physiologischer Salzlösung beträgt bei den Polyurethan-Schäumen typischerweise 100 bis 2000 %, bevorzugt 300 bis 2000 %, besonders bevorzugt 800 bis 2000 % und ganz besonders bevorzugt bei 1000 bis 1800 % (Masse der aufgenommenen Flüssigkeit bezogen auf die Masse des trockenen Schaums; Bestimmung nach DIN EN 13726-1, Teil 3.2). Im Vergleich zu anderen hydrophilen Schäumen kann mit den erfindungsgemäßen Polyurethan-Schäumen auch ohne die Verwendung von superabsorbierenden Polymeren eine sehr hohe Absorption von physiologischer Salzlösung erreicht werden. Selbstverständlich ist die Einarbeitung von Superabsorbern aber auch bei den erfindungsgemäßen Polyurethan-Schäumen möglich.

Die Polyurethan-Schäume weisen eine gute mechanische Festigkeit und hohe Elastizität auf. Typischerweise sind die Werte für die Zugfestigkeit größer als 40 kPa, für die Bruchdehnung größer als 30 % und für die Rückprallelastizität größer als 60 %. Bevorzugt ist die Zugfestigkeit größer als 50 kPa, die Bruchdehnung größer als 40 % und die Rückprallelastizität größer als 80 % (Bestimmung nach DIN 53504, DIN 53455, DIN EN ISO 3386-1).

Nach der Herstellung können die Polyurethan-Schäume nach an sich bekannten Verfahren zu flächigen Materialien verarbeitet werden, welche dann beispielsweise als Bestandteil einer Wundauflage, eines kosmetischen Artikels oder eines Inkontinenzprodukts eingesetzt werden. In der Regel werden dazu Blockschäume auf die gewünschte Dicke nach gängigen Methoden geschnitten und flächige Materialien mit einer Dicke von typischerweise 10 µ bis 5 cm, bevorzugt 0,1 mm bis 1 cm, besonders bevorzugt 0,1 mm bis 6 mm, ganz besonders bevorzugt 0,2 mm bis 6 mm zu erhalten.

Durch geeignete Gießtechniken können die beschriebenen flächigen Materialien aber auch direkt durch Auftrag und Aufschäumen der erfindungsgemäßen Zusammensetzung auf ein Substrat, z.B. ein gegebenenfalls vorbehandeltes Papier oder Textil, erhalten werden.

Die Polyurethan-Schäume enthalten einen nur geringen mit Wasser extrahierbaren Anteil von maximal 2 Gew.%, bevorzugt maximal 1 Gew.-%, d.h. sie enthalten nur sehr geringe Mengen an chemisch nicht gebundenen Bestandteilen.

Die Polyurethan-Schäume können überdies mit weiteren Materialien verklebt, laminiert oder beschichtet werden, beispielsweise auf Basis von Hydrogelen, (semi-) permeablen Folien, Schaumfolien, Beschichtungen, Hydrokolloiden oder anderen Schäumen.

Die erfindungsgemäßen Polyurethan-Schäume sind besonders geeignet zur Herstellung von Wundauflagen. Dabei können die Polyurethan-Schäume in direktem oder indirektem Kontakt mit der Wunde sein. Bevorzugt werden die Polyurethan-Schäume jedoch in direktem Kontakt mit der Wunde eingesetzt, um beispielsweise eine optimale Absorption von Wundflüssigkeit zu gewährleisten. Die Polyurethan-Schäume zeigen keine Zelltoxizität (Bestimmung nach ISO 10993-5 und ISO 10993-12).

Die Polyurethan-Schäume, welche als Wundauflage eingesetzt werden, müssen zusätzlich noch in einem weiteren Verfahrensschritt sterilisiert werden. Zur Sterilisation kommen die dem Fachmann an sich bekannten Verfahren zum Einsatz, bei denen eine Sterilisation durch thermische Behandlung, chemische Stoffe wie Ethylenoxid oder Bestrahlung beispielsweise durch Gammabestrahlung erfolgt. Die Bestrahlung kann dabei gegebenenfalls unter Schutzgasatmosphäre erfolgen. Die erfindungsgemäßen Polyurethan-Schäume haben dabei den großen Vorteil, dass sie sich bei Bestrahlung, insbesondere bei Bestrahlung mit Gammastrahlen, nicht verfärben.

Ebenfalls möglich ist die Zugabe, Einarbeitung oder Beschichtung von bzw. mit antimikrobiellen oder biologischen Wirkstoffen, welche sich beispielsweise in Bezug auf die Wundheilung und die Vermeidung von Keimbelastungen positiv auswirken.

### Beispiele

Sofern nicht abweichend gekennzeichnet, beziehen sich alle Prozentangaben auf das Gewicht. Die Bestimmung der Festkörpergehalte erfolgte nach DIN-EN ISO 3251. Die Bestimmung der Viskositäten erfolgte bei 23 °C und wurde nach DIN 53019 durchgeführt. Die NCO-Gehalte wurden volumetrisch gemäß DIN-EN ISO 11909 bestimmt.

**Verwendete Substanzen und Abkürzungen:**
- Carboxylat 1:: 10 % Natriumoleat in Wasser
- Carboxylat 2:: 10 % Natrium-2-Ethylhexanoat in Wasser
- Dispergiermittel EM:: Polyetherpolyol der OH-Zahl 70 mg KOH/g (Rhein Chemie Rheinau GmbH, Mannheim, DE)
- Zusatzmittel VP.PU 3240:: Polyglykolester der OH-Zahl 100 mg KOH/g (Rhein Chemie Rhei-nau GmbH, Mannheim, DE)
- Tegostab^{®} B 2370:: Polysiloxan-polyoxyalkylen Blockcopolymer (Degussa-Goldschmidt AG, Essen, DE)
- Desmodur^{®} N 3400:: Aliphatisches Polyisocyanat (HDI-Uretdion), NCO-Gehalt 21,8 %
- Desmodur^{®} N 3600:: Aliphatisches Polyisocyanat (HDI-Isocyanurat), NCO-Gehalt 24 %
- Pluronic^{®} PE 3500:: EO/PO-Blockcopolymer (BASF, Ludwigshafen, DE)
- Pluronic^{®} PE 6800:: EO/PO-Blockcopolymer (BASF, Ludwigshafen, DE)
- Desmopheno^{®} 41 WB01:: Polyetherpolyol der OH-Zahl 37 mg KOH/g (Bayer MaterialScience AG, Leverkusen, DE)
- Polyether PW 56:: Polyetherpolyol der OH-Zahl 56 mg KOH/g (Bayer MaterialScience AG, Leverkusen, DE)
- Polyether PEG 400:: Polyetherpolyol der OH-Zahl 280 mg KOH/g (BASF AG, Ludwigs-hafen, DE)
- Polyether LB 25:: monofunktioneller Polyether auf Ethylenoxid-/Propylenoxidbasis, zahlenmittleres Molekulargewicht 2250 g/mol, OH-Zahl 25 mg KOH/g (Bayer MaterialScience AG, Leverkusen, DE)

### Beispiel 1: Herstellung des Polyurethan-Präpolymers 1

Zu einem Gemisch aus 1000g HDI und 1g Benzoylchlorid wurde bei 80°C innerhalb von 3h 1000g eines Polyalkylenoxids mit einer Molmasse von 4680 g/mol gestartet auf Glycerin, einem Ethylenoxidgewichtsanteil von 72 % und Propylenoxidgewichtsanteil von 28 %, das zuvor bei 100°C während 6h bei einem Druck von 0,1 mbar getrocknet wurde, zugetropft und für 12h nachgerührt. Das überschüssige HDI wurde durch Dünnschichtdestillation bei 130°C und 0,1 mbar entfernt, wobei die nicht flüchtigen Bestandteile mit 1g Chlorpropionsäure stabilisiert wurden. Man erhielt ein Präpolymer mit einem NCO-Gehalt von 2,77 % und einer Viskosität von 3500 mPas.

### Beispiel 2: Herstellung des Polyurethan-Präpolymers 2

Zu einem Gemisch aus 200g HDI, 1g Benzoylchlorid und 1 g Methyltosylat wurde bei 80°C innerhalb von 2h 400g eines Polyalkylenoxids mit einer Molmasse von 5800 g/mol gestartet auf Glycerin, einem Ethylenoxidgehalt von 80 % und einem Propylenoxidgehalt von 20 %, das vorher bei 100°C während 6h bei einem Druck von 0,1 mbar getrocknet wurde, zugetropft und für 12h nachgerührt. Das überschüssige HDI wurde durch Dünnschichtdestillation bei 130°C und 0,1 mbar entfernt. Man erhielt ein Präpolymer mit einem NCO-Gehalt von 2,31 % und einer Viskosität von 6070 mPas.

### Beispiel 3: Herstellung des Polyurethan-Präpolymers 3

Zu einem Gemisch aus 1440g HDI und 4g Benzoylchlorid wurde bei 80°C innerhalb von 2h 2880g eines Polyalkylenoxids mit einer Molmasse von 4680 g/mol gestartet auf Glycerin, einem Ethylenoxidgewichtsanteil von 72 % und Propylenoxidgewichtsanteil von 28 %, das zuvor bei 100°C während 6h bei einem Druck von 0,1 mbar getrocknet wurde, zugetropft und für 1h nachgerührt. Das überschüssige HDI wurde durch Dünnschichtdestillation bei 130°C und 0,1 mbar entfernt. Man erhielt ein Präpolymer mit einem NCO-Gehalt von 2,11 % und einer Viskosität von 3780 mPas.

### Beispiel 4: Herstellung des Polyurethan-Präpolymers 4

Zu einem Gemisch aus 200g IPDI, 1g Benzoylchlorid und 1g Methyltosylat wurde bei 80°C innerhalb von 2h 400g eines Polyalkylenoxids mit einer Molmasse von 5800 g/mol gestartet auf Glycerin, einem Ethylenoxidgehalt von 80 % und einem Propylenoxidgehalt von 20 %, das vorher bei 100°C während 6h bei einem Druck von 0,1 mbar getrocknet wurde, zugetropft und für 12h nachgerührt. Das überschüssige IPDI wurde durch Dünnschichtdestillation bei 130°C und 0,1 mbar entfernt. Man erhielt ein Präpolymer mit einem NCO-Gehalt von 2,36 % und einer Viskosität von 8800 mPas.

### Beispiele 5-13: Herstellung von Schaumstoffen aus den Polyurethan-Präpolymeren 1-3

Die beiden Isocyanat-Komponenten wurden 15 Sekunden mit einer Rührerdrehzahl von 1200 Upm homogenisiert, dann die weiteren Komponenten eingewogen, weitere 10 Sekunden verrührt und in einen Becher vom Volumen 500 ml eingebracht.

| **Komponente [g]** | **Beispiel** | | | | | | | | |
|---|---|---|---|---|---|---|---|---|---|
| | **5** | **6** | **7** | **8** | **9** | **10** | **11** | **12** | **13** |
| Präpolymer | 36,0¹⁾ | 36,0²⁾ | 36,0²⁾ | 36,0²⁾ | 36,0²⁾ | 20,0²⁾ | 20,0³⁾ | 20,0²⁾ | 20,0²⁾ |
| Oligomer | 4,0⁴⁾ | 4,0⁴⁾ | 4,0⁴⁾ | 4,0⁴⁾ | 4,0⁴⁾ | 2,2⁵⁾ | 2,2⁴⁾ | | 2,2⁴⁾ |
| Additiv | 0,6⁶⁾ | 0,6⁶⁾ | 1,2⁷⁾ | | 0,6⁸⁾ | | 0,4⁹⁾ | 0,4⁹⁾ | 0,4⁹⁾ |
| DBU | 0,05 | 0,05 | 0,05 | 0,05 | 0,05 | 0,03¹⁰⁾ | 0,03 | 0,03¹⁰⁾ | 0,03¹⁰⁾ |
| Carboxylat | 2,0¹¹⁾ | 2,0¹²⁾ | 2,0¹¹⁾ | 2,0¹¹⁾ | 2,0¹¹⁾ | 1,1¹¹⁾ | 1,1¹¹⁾ | 1,1¹¹⁾ | 1,1¹¹⁾ |
| | | | | | | | | | |
| Startzeit [s] | 7 | 35 | 30 | 15 | 26 | 28 | 20 | 30 | 20 |
| Rohdichte [g/cm³] | 0,12 | 0,12 | 0,12 | 0,12 | 0,13 | 0,14 | 0,17 | 0,27 | 0,13 |

| | | | | | | | | | |
|---|---|---|---|---|---|---|---|---|---|
| ¹⁾ Präpolymer 2; ²) Präpolymer 1; ³⁾ Präpolymer 3; ⁴⁾ Desmodur N 3400; ⁵⁾ Desmodur N 3600; ⁶⁾ Dispergiermittel EM; ⁷⁾ Zusatzmittel VP.PU 3240/Tegostab^{®} B 2370 (je 50 %); ⁸⁾ Pluronic^{®} PE 3500; ⁹⁾ Pluronic^{®} PE 6800; ¹⁰⁾ gelöst in 0,5 g Desmophen^{®} 41WB01; ¹¹⁾ Carboxylat 1; ¹²⁾ Carboxylate 2 | | | | | | | | | |

In den Beispielen 5 bis 13 wurden Schaumstoffe von regelmäßig feiner Zellstruktur erhalten, die dimensionsstabil und elastisch sind. Sie zeigen nach Belastung ein sehr starkes Rückstellvermögen und einen geringen Druckverformungsrest mit einer relativ niedrigen Stauchhärte von 1-5 kPa bei 40 % Stauchung bei höheren Rohdichten. Dies ist wichtig für eine gute Aufnahmekapazität an Wundsekret und der Anpassung an Konturen. Exemplarisch wurde gemäß Richtlinie ISO 10993.5 gezeigt, dass der aus Beispiel 13 resultierende Schaum als nicht zytotoxisch einzustufen ist.

Wie Beispiel 12 zeigt, resultieren bei Verzicht auf die heterocyclischen Oligomere B) kompaktere Polyurethanschäume.

### Vergleichsbeispiel 1: Carboxylatfreie Umsetzung der Präpolymere

Unter vergleichbaren Bedingungen wie in den Beispielen 5-13 wurden zunächst 20,0 g Präpolymer 1 und 2,2 g Desmodur N 3400 homogenisiert und anschließend mit einer Lösung von 0,03 g DBU in 1,0 g Wasser versetzt. Nach einer Startzeit von 20 Sekunden begann die Schaumexpansion, jedoch erleidete der resultierende Schaum starken Schrumpf. Auch die Zugabe von an sich bekannten Schaumhilfsmitteln wie beispielsweise Pluronic^{®} PE 6800 zur wässrigen Lösung des Katalysators verhindert diesen Schrumpf nicht.

Wie dieses Vergleichsbeispiel verdeutlicht, stellen die erfindungsgemäßen Carboxylate eine maßgebliche Komponente in der beschriebenen Schaumherstellung dar: Ohne diese Salze kommt es nach dem Aufschäumen der Polyurethanschäume - auch trotz Zugabe allgemein hin bekannter Schaumadditive - zu starkem Schrumpf, was produktionstechnisch nicht bevorzugt ist. Ebenso wird bei der Verwendung entsprechender Alkali-Sulfate oder -Phosphate starker Schrumpf bei den resultierenden Polyurethanschäumen beobachtet.

### Beispiele 14-17: Herstellung von Schaumstoffen aus Polvurethan-Präpolymer 1

20,0 g Präpolymer 1 und 2,2 g Desmodur^{®} N 3400 wurden 15 Sekunden mit einer Rührerdrehzahl von 1200 Upm homogenisiert, dann 0,03 g DBU gelöst in 0,5 g Desmopheno^{®} 41WB01, 1,1 g Carboxylat 1 und 0,2 g der Alkoholkomponente eingewogen, weitere 10 Sekunden verrührt und in einen Becher vom Volumen 250 ml eingebracht.

| | **Beispiel** | | | |
|---|---|---|---|---|
| | **14** | **15** | **16** | **17** |
| Alkohol | 1,4-Butandiol | Polyether PEG 400 | Polyether PW 56 | Polyether LB 25 |
| Startzeit [s] | 20 | 21 | 20 | 30 |
| Rohdichte [g/cm³] | 0,13 | 0,13 | 0,12 | 0,16 |

Wie die Beispiele 14 bis 17 verdeutlichen, wurden auch nach Abmischung der erfindungsgemäßen wässrigen Carboxylate mit Diolen Schaumstoffe von regelmäßig feiner Zellstruktur erhalten, die dimensionsstabil und elastisch sind.

### Beispiele 18-24: Herstellung von Schaumstoffen aus Polyurethan-Präpolymer 1

20,0 g Präpolymer 1 und 2,2 g Desmodur^{®} N 3400 wurden 15 Sekunden mit einer Rührerdrehzahl von 1200 Upm homogenisiert, dann 0,03 g Katalysator und 1,1 g Carboxylat 1 eingewogen, weitere 10 Sekunden verrührt und in einen Becher vom Volumen 250 ml eingebracht.

| | **Beispiel** | | | | | | |
|---|---|---|---|---|---|---|---|
| | **18** | **19** | **20** | **21** | **22** | **23** | **24**¹⁾ |
| Katalysator | DBU | DABCO | SO | ZO | TMG | - | - |
| Startzeit [s] | 20 | 40 | 30 | 60 | 40 | 50 | 120 |
| Rohdichte [g/cm³] | 0,13 | 0,11 | 0,13 | 0,13 | 0,16 | 0,13 | 0,11 |
| Zellstruktur | fein | fein | fein | sehr grob | fein | mittel | fein |

| | | | | | | | |
|---|---|---|---|---|---|---|---|
| ¹⁾ Carboxylat 1 ersetzt durch 1,0 g einer 2%igen wässrigen Natriumoleat-Lösung | | | | | | | |

Wie die Beispiele 18 bis 22 verdeutlichen, konnte mit der Wahl des Katalysators neben der Reaktionsgeschwindigkeit auch Einfluss auf die Zellstruktur genommen werden. Zudem verdeutlichen die Beispiele 23 und 24, dass Carboxylat 1 auch katalytische Eigenschaften besitzt.

### Beispiel 25: Herstellung eines Schaumstoffs aus dem Polyurethan-Präpolymer 4

Zur Ermittlung von Stauchhärte nach DIN 53577 und der Rohdichte nach DIN 53420 wurden in einem 1000 ml Polypropylen-Gefäß 108 g Präpolymer 4 und 12 g Desmodur N 3400 für 15 Sekunden mit einer Rührerdrehzahl von 1200 Upm homogenisiert. Anschließend wurden 1,8 g Dispergiermittel EM, 0,15 g DBU und 3 g Carboxylat 1 zugesetzt und das Gemenge für weitere 10 Sekunden verrührt. Der resultierende, ausgehärtete Schaum zeigte keine große Haftung an der Gefäßwandung. Er besaß bei 40 % Stauchung eine Stauchhärte von 3,1 kPa sowie eine Rohdichte von 0,08 g/cm³.

### Beispiel 26: (Bestimmung der Extraktmenge von Polyurethanschaum 5

10 g des Schaums aus Beispiel 5 wurden für 48 Stunden in 300 ml vollentsalztes Wasser bei 36°C eingelegt und durch Titration des chemischen Sauerstoffbedarfs gemäß DIN EN 1484 die Extraktmenge bestimmt. Sie betrug 0,6 Gew.-%.

### Beispiel 27: (Bestimmung der Extraktmenge von Polyurethanschaum 8

4,7 g des Schaums aus Beispiel 8 wurden für 7 Tage in 220 ml vollentsalztes Wasser bei 37°C eingelegt und durch Titration des chemischen Sauerstoffbedarfs gemäß DIN EN 1484 die Extraktmenge bestimmt. Sie betrug 0,2 Gew.-%.

Beispiel 24 der US 5065752, das einzige Beispiel, in dem zumindest eine Teilmenge des Isocyanats aliphatisch war, beschreibt einen Schaum mit einem Extraktgehalt von 30 Gew.-%.

## Patentansprüche

1. Verfahren zur Herstellung hydrophiler, aliphatischer Polyurethan-Schäume, bei dem Zusammensetzungen umfassend
A) isocyanatfunktionelle Präpolymere mit einem Gewichtsanteil an niedermolekularen, aliphatischen Diisocyanaten mit einer Molmasse von 140 bis 278 g/mol von unter 1,0 Gew.-% bezogen auf das Präpolymer, erhältlich durch Umsetzung von
A1) niedermolekularen, aliphatischen Diisocyanaten einer Molmasse von 140 bis 287 g/mol mit
A2) di- bis hexafunktionellen Polyalkylenoxiden einer OH-Zahl von 22,5 bis 112 mg KOH/g, und einem Ethylenoxidanteil von 50 bis 100 mol% bezogen auf die Gesamtmenge der enthaltenen Oxyalkylengruppen,
B) gegebenenfalls heterocyclische, 4-Ring- oder 6-Ring-Oligomere von niedermolekularen, aliphatischen Diisocyanaten mit einer Molmasse von 140 bis 278 g/mol,
C) Wasser,
D) gegebenenfalls Katalysatoren,
E) C₈- bis C₂₂-Monocarbonsäuren oder deren Ammonium- oder Alkalisalze oder C₁₂- bis C₄₄-Dicarbonsäuren oder deren Ammonium- oder Alkalisalze,
F) gegebenenfalls Tenside und
G) gegebenenfalls ein- oder mehrwertige Alkohole
bereitgestellt, aufgeschäumt und ausgehärtet werden.

2. Verfahren gemäß Anspruch 1, **dadurch gekennzeichnet, dass** der NCO-Gehalt der isocyanatfunktionellen Präpolymere A) 1,5 bis 3,0 Gew.-% beträgt.

3. Verfahren gemäß Anspruch 1 oder 2, **dadurch gekennzeichnet, dass** in A1) ausschließlich HDI, IPDI oder deren Mischungen untereinander eingesetzt werden.

4. Verfahren gemäß einem der Ansprüche 1 bis 3, **dadurch gekennzeichnet, dass** in A2) als Polyalkylenoxide Copolymere aus Ethylenoxid und Propylenoxid mit einem Ethylenoxidgehalt, bezogen auf die Gesamtmenge der enthaltenen Oxyalkylengruppen, von 60 bis 85 mol%, gestartet auf Polyolen oder Aminen eingesetzt werden.

5. Verfahren gemäß einem der Ansprüche 1 bis 4, **dadurch gekennzeichnet, dass** die Polyalkylenoxide der Komponente A2) zahlenmittlere Molekulargewichte von 3000 bis 8500 g/mol aufweisen.

6. Verfahren gemäß einem der Ansprüche 1 bis 5, **dadurch gekennzeichnet, dass** die Polyalkylenoxide der Komponente A2) OH-Funktionalitäten von 3 bis 4 aufweisen.

7. Verfahren gemäß einem der Ansprüche 1 bis 6, **dadurch gekennzeichnet, dass** in Komponente B) heterocyclische 4-Ring-Oligomere eingesetzt werden.

8. Verfahren gemäß einem der Ansprüche 1 bis 7, **dadurch gekennzeichnet, dass** als Katalysatoren der Komponente D) Metallsalze, Amine, Amidine und Guanidine eingesetzt werden.

9. Verfahren gemäß einem der Ansprüche 1 bis 7, **dadurch gekennzeichnet, dass** die Komponenten A) bis E) in den folgenden Mengen eingesetzt werden:
100 Gewichtsteile isocyanatfunktioneller Präpolymere A)
5 bis 15 Gewichtsteile heterocyclischer Oligomere B)
1 bis 10 Gewichtsteile Wasser C)
0,1 bis 0,5 Gewichtteile an Katalysatoren D)
0,1 bis 1 Gewichtteile an C₈- bis C₁₂-Monocarbonsäuren oder deren Ammonium- oder Alkalisalze oder C₁₂- bis C₄₄-Dicarbonsäuren oder deren Ammonium- oder Alkalisalze als Komponente E)

10. Zusammensetzungen umfassend
A) isocyanatfunktionelle Präpolymere mit einem Gewichtsanteil an niedermolekularen, aliphatischen Diisocyanaten mit einer Molmasse von 140 bis 278 g/mol von unter 1,0 Gew.-% bezogen auf das Präpolymer, erhältlich durch Umsetzung von
A1) niedermolekularen, aliphatischen Diisocyanaten einer Molmasse von 140 bis 287 g/mol mit
A2) di- bis hexafunktionellen, bevorzugt tri- bis hexafunktionellen Polyalkylenoxiden einer OH-Zahl von 22,5 bis 112, bevorzugt von 31,5 bis 56, und einem Ethylenoxidanteil von 50 bis 100 mol%, bevorzugt 60 bis 85 mol%, bezogen auf die Gesamtmenge der enthaltenen Oxyalkylengruppen,
B) gegebenenfalls heterocyclische, 4-Ring- oder 6-Ring-Oligomere von niedermolekularen, aliphatischen Diisocyanaten mit einer Molmasse von 140 bis 278 g/mol,
C) Wasser,
D) gegebenenfalls Katalysatoren,
E) C₈- bis C₂₂-Monocarbonsäuren oder deren Ammonium- oder Alkalisalze oder C₁₂- bis C₄₄-Dicarbonsäuren oder deren Ammonium- oder Alkalisalze,
F) gegebenenfalls Tenside und
G) gegebenenfalls ein- oder mehrwertige Alkohole
bereitgestellt, aufgeschäumt und ausgehärtet werden.

11. Polyurethanschäume erhältlich nach einem Verfahren gemäß einem der Ansprüche 1 bis 9 oder aus Zusammensetzungen gemäß Anspruch 10.

12. Wundauflagen, kosmetische Artikel oder Inkontinenzprodukte erhältlich unter Verwendung von Polyurethanschäumen gemäß Anspruch 11.
